# EUROPEAN PATENT SPECIFICATION

(11) **EP 1 781 291 B1**
(45) Date of publication and mention of the grant of the patent: **03.11.2010**
(21) Application number: 05754710.1
(22) Date of filing: 01.06.2005
(51) Int. Cl.: A61K 31/485, A61P 25/14

(54) **OPIOIDS FOR THE TREATMENT OF THE RESTLESSNESS OF THE LOWER LIMBS**
OPIOIDE ZUR BEHANDLUNG VON RUHELOSIGKEIT IN DEN UNTEREN GLIEDMASSEN
OPIOIDES POUR LE TRAITEMENT DES IMPATIENCES DES MEMBRES INFÉRIEURS

(30) Priority: 08.06.2004 EP 04013469
(43) Date of publication of application: 09.05.2007
(60) Divisional application: 10175878.7
(73) Proprietor: EURO-CELTIQUE S.A., 1653 Luxembourg (LU)
(72) Inventor: FLEISCHER, Wolfgang, 55218 Ingelheim (DE); REIMER, Karen, 65582 Hambach (DE); GAWORA, Karin, 35080 Bad Endbach (DE)
(74) Representative: Ehlich, Eva Susanne
(86) International application number: PCT/EP2005/005888
(87) International publication number: WO 2005/120506

(56) References cited:
- WO-A-98/25613
- WO-A-02/092059
- WO-A-03/007802
- WO-A-03/013538
- A.S.WALTERS AND ALL.: "Succesful Treatment of the Idiopathic Restless Legs Syndrome in a Randomized Double-Blind Trial of Oxycodone Versus Placebo" SLEEP, vol. 16, no. 4, 1993, pages 327-332, XP008034509 US

## Description

The invention concerns the treatment of the restless leg syndrome (RLS). In particular, the invention concerns the use of opioid sustained release oral dosage forms for the manufacture of preparations for the treatment of RLS. The preparations can be used to treat insomnia and disorders of sleep associated with RLS.

### Background of the invention

Restless legs syndrome (RLS) is a neurological disorder characterized by unpleasant sensations in the legs and an uncontrollable urge to move when at rest, in an effort to relieve these feelings. RLS sensations are often described by people as burning, creeping, tugging, or like insects crawling inside the legs. Often called paresthesias (abnormal sensations) or dysesthesias (unpleasant abnormal sensations), the sensations range in severity from uncomfortable to irritating to painful.

The most distinctive or unusual aspect of the condition is that lying down and trying to relax activates the symptoms. As a result, most people with RLS have difficulty falling asleep and staying asleep. Left untreated, the condition causes exhaustion and daytime fatigue. Many people with RLS report that their job, personal relations, and activities of daily living are strongly affected as a result of their exhaustion. They are often unable to concentrate, have impaired memory, or fail to accomplish daily tasks.

RLS occurs in both genders, although the incidence may be slightly higher in women. Although the syndrome may begin at any age, even as early as infancy, most patients who are severely affected are middle-aged or older. In addition, the severity of the disorder appears to increase with age. Older patients experience symptoms more frequently and for longer periods of time.

More than 80 percent of people with RLS also experience a more common condition known as periodic limb movement disorder (PLMD). PLMD is characterized by involuntary leg twitching or jerking movements during sleep that typically occur every 10 to 60 second, sometimes throughout the night. The symptoms cause repeated awakening and severely disrupted sleep. Unlike RLS, the movements caused by PLMD are involuntary - people have no control over them. Although most patients with RLS also develop PLMD, people with PLMD do not generally develop RLS. Like RLS, the cause of PLMD is unknown. Within the meaning of the present invention "the treatment of RLS" means also "the treatment of RLS and/or PLMD".

As described above, people with RLS feel uncomfortable sensations in their legs, especially when sitting or lying down, accompanied by an irresistible urge to move about. These sensations usually occur deep inside the leg, between the knee and ankle; more rarely, they occur in the feet, thighs, arms, and hands. Although the sensations can occur on just one side of the body, they most often affect both sides.

Because moving the legs (or other affected parts of the body) relieves the discomfort, people with RLS often keep their legs in motion to minimize or prevent the sensations. They may pace the floor, constantly move their legs while sitting, and toss and turn in bed.

Most people find the symptoms to be less noticeable during the day and more pronounced in the evening or at night, especially during the onset of sleep. For many people, the symptoms disappear by early morning, allowing for more refreshing sleep at that time. Other triggering situations are periods of inactivity such as long car trips, sitting in a movie theatre, long distance flights, immobilization in a cast, or relaxation exercises.

The symptoms of RLS vary in severity and duration from person to person. Mild RLS occurs episodically, with only mild disruption of sleep onset, and causes little distress. In moderately severe cases, symptoms occur only once or twice a week but result in significant delay of sleep onset, with some disruption of daytime function. In severe cases of RLS, the symptoms occur more than twice a week and result in burdensome interruption of sleep and impairment of daytime function.

Symptoms may begin at any stage of life, although the disorder is more common with increasing age. Sometimes people will experience spontaneous improvement over a period of weeks or months. Although rare, spontaneous improvement over a period of years also can occur. If these improvements occur it is usually during the early stages of the disorder. In general, however, symptoms become more severe over time.

Generally dopaminergic agents, largely used to treat Parkinson's disease, have been shown to reduce RLS symptoms and PLMD and are considered the initial treatment of choice. Benzodiazepines (such as clonazepam and diazepam) may be prescribed for patients who have mild or intermittent symptoms. These drugs help obtain a more restful sleep but they do not fully alleviate RLS symptoms and can cause daytime sleepiness.

Anticonvulsants such as carbamazepine and gabapentin are also useful for some patients, as they decrease the sensory disturbances (creeping and crawling sensations). Dizziness, fatigue, and sleepiness are among the possible side effects.

Opioids have been suggested for the treatment of RLS (see A.S. Walters et al., Sleep, Vol. 16, 1993, p. 327-332). However, there is still a need for preparations containing opioids with improved patient compliance and which are advantageous for constant medication. Long term compliance is highly desirable.

### Objects and summary of the invention

It is an object of the invention to provide an opioid oral dosage form with a prolonged duration of action, preferably at least 12 hours, more preferred at least 24 hours for the treatment of moderate to severe RLS symptoms, preferably severe RLS symptoms.

It is a further object of the invention to provide opioid preparations as outlined above, which cause less side effects such as respiratory depression and obstipation and which are provided with abuse-preventing characteristics.

The invention further comprises a use of sustained release oral dosage forms for the manufacture of a medicament for treating patients with RLS symptoms as claimed.

The scope of the invention is limited by the appended claims.

### Detailed description of the invention

In the context of the invention, the term "opioid composition" or "opioid" or "active" is used interchangeable and is considered to include opioid agonists and opioid antagonists and mixed opioid antagonists/agonists as well as mixtures thereof.

The preparations according to the present invention comprise a mixture or an opioid agonist and an opioid antagonist.

In the context of the invention the term "slow release formulations or dosage formes" "retard formulations or dosage forms" or "sustained release formulations or dosage forms" or "formulations or dosage forms with prolonged duration of action" are used interchangeable and are understood to be formulations or dosage forms that exhibit a prolonged release profile for the active incorporated and which provide a sufficient therapeutic effect for at least 12 hours at steady state.

The invention is premised on the fact that opioid agonists are useful for the treatment of RLS /PLMD-symptoms such as unpleasant sensations in the legs and an uncontrollable urge to move when at rest the difficulties with lying down and trying to relax as well as associated difficulties in falling asleep and/or staying asleep. In particular, the use of opioid sustained release oral dosage forms results in better patient compliance and renders the patient on constant medication more independent from taking medication during day and night time. The minimum of the therapeutically necessary drug can be administered, thereby reducing side effects and the risk of addiction. In particular, combinations of opioid agonists and antagonist are advantageous with respect to reduced side effects and additionally reduce the risk of abuse.

### ACTIVE INGREDIENTS

According to the invention, opioid agonists comprise all compounds that belong to class NO2A of opioid analgesics according to the ATC Classification of the WHO, and that display a therapeutic effect upon application in accordance with the invention. Preferably, an opioid agonist is selected from the group of morphine, oxycodone, hydromorphone, propoxyphene, nicomorphine, dihydrocodeine, diamorphine, papaveretum, codeine, ethylmorphine, phenylpiperidine and derivates thereof, methadone, dextropropoxyphene, buprenorphine, pentazocine, tilidine, tramadol and hydrocodone. Further examples for useable analgesics according to the invention are meperidine, oxymorphone, alphaprodine, anileridine, dextromoramide, metopone, levorphanol, phenazocine, etoheptazine, propiram, profadol, phenampromide, thiambuten, pholcodeine, codeine, dihydrocodeinon, fentanyl, 3-trans-dimethylamino-4-phenyl-4-trans-carbethoxy-A'-cyclohexen, 3-dimethylamino-0-(4-methoxyphenyl-carbamoyl)-propiophenone oxime, (-)β-2'-hydroxy-2, 9-dimethyl-5-phenyl-6, 7-benzomorphane, (-)2'-hydroxy-2-(3-methyl-2-butenyl)-9-methyl-5-phenyl-6, 7-benzomorphane, pirinitramide, (-)α-5,9-diethyl-2' hydroxy-2-methyl-6, 7-benzomorphane, ethyl 1-(2-dimethylaminoethyl)-4,5,6,7-tetrahydro-3-methyl-4-oxo-6-phenyl-indol-2-carboxylate,1-benzoylmethyl-2, 3-dimethyl-3- (m-hydroxy-phenyl) -piperidine, N-allyl-7α (1-R-hydroxy-1-methylbutyl)-6,14-endo-ethanotetrahydronororipavine, (-)2'-hydroxy-2-methyl-6,7-benzomorphane, noracylmethadol, phenoperidine, α-d1-methadol, α -1-methadol, β-d1-acetylmethadol, α-1-acetylmethadol and β-1-acetylmethadol.

Especially preferred analgesically effective opiod agonists are oxycodone, hydrocodone, hydromorphone, morphine, methadone, oxymorphone, fentanyl and sufentanyl. More preferred embodiments contain oxycodone or morphine.

According to the invention, antagonists comprise such compounds that counteract opioid agonists (as defined earlier). Such compounds can also be found in the ATC Classification of the WHO. According to the invention, compounds are preferred that upon application in accordance with the invention decrease the side effects, the habituation effects and the addictive potential caused by the opioid agonists. Antagonists can comprise among others, naltrexone, naloxone, nalmefene, nalorphine, nalbuphine, naloxoneazinen, methylnaltrexone, ketylcyclazocine, norbinaltorphimine, naltrindol, 6-β-naloxol and 6-β-naltrexol.

Especially preferred antagonists comprise naltrexone, nalmefene and naloxone. More preferred embodiments comprise naloxone.

Especially preferred embodiments of the invention comprise the combination of oxycodone and naloxone in a sustained release oral dosage form. Preferably, oxycodone is present in excess to the unit dosage amount of naloxone.

In the case of oxycodone and naloxone, preferred weight ratios of agonist to antagonist lie within a weight ratio range of 25:1 at maximum, preferably of 20:1 at maximum, especially preferred are the weight ratio ranges 15:1 and 10:1 and more preferred 5:1, 4:1, 3:1, 2:1 and 1:1.

The absolute amounts of agonist and antagonist to be used depend on the choice of the active compounds. Preferably the agonist and antagonist are released from the pharmaceutical preparation only in an independent and invariant manner.

If oxycodone and naloxone are used for a combination preparation, preferably between 10 and 150 mg, especially preferably between 10 and 80 mg of oxycodone (typical amounts for use) and preferably between 1 and 50 mg naloxone per unit dosage are used.

In other preferred embodiments of the invention, the preparations may comprise between 5 and 50 mg of oxycodone, between 10 and 40 mg of oxycodone, between 10 and 30 mg of oxycodone or approximately 20 mg of oxycodone. Preferred embodiments of the invention may also comprise preparations with between 1 and 40 mg naloxone, 1 and 30 mg naloxone, 1 and 20 mg naloxone or between 1 and 10 mg naloxone per unit dosage.

Preferably, the ratio of oxycodone and naloxone has to be chosen in such a way that the appropriate release profiles for both active substances are guaranteed and that the agonist can display its therapeutic effect while the amount of the antagonist is chosen in such a way that habituation- or addiction-promoting effects and side effects of the agonist are reduced or abolished, without (substantially) affecting the therapeutic effect of the agonist. According to the invention, development of habituation and addiction as well as obstipation and breath depression are to be considered as side effects of therapeutically effective opioid agonists.

In the context of this invention, all kinds of pharmaceutically acceptable salts and derivatives (including prodrugs) of the active ingredient may be used instead or together with the active unmodified ingredient, in amounts equivalent to the amount of unmodified active ingredient as indicated herein.

Oxycodone and naloxone may be present as their hydrochloride, sulphate, bisulfate, tatrate, nitrate, citrate, bitartrate, phosphate, malate, maleate, hydrobromide, hydroiodide, fumarate or succinate.

### DOSAGE FORMS

The opioid is provided in an oral dosage form. The oral dosage form is designed as a sustained release preparation or it is a combined immediate release and sustained release oral dosage form. The dosage form may thus e.g. comprise a sustained release portion outwardly coated with an immediate-release formulation. The active ingredient may be the same, or may be different in these different portions.

In certain embodiments, the oral dosage forms of the present invention comprise an opioid combined with excipients, i.e., pharmaceutically acceptable organic or inorganic carrier substances suitable for oral administration which are known in the art. Suitable pharmaceutically acceptable carriers include water salt solutions, alcohols, gum arabic, vegetable oils, benzyl alcohols, polyethylene glycols, gelate, carbohydrates such as lactose, amylose or starch, magnesium stearate talc, silicic acid, viscous paraffin, perfume oil, digestible long chain substituted or unsubstituted hydrocarbons such as fatty acid monoglycerides and diglycerides, pentaerythritol fatty acid esters, hydrophilic or hydrophobic polymers, such as cellulose and cellulose derivatives, such as alkylcellulose or hydroxyalkylcellulose, acrylic resins, such as the polymers known under the Eudragit^{(R)} trade name as well as polyvinylpyrrolidone. The pharmaceutical compositions can be sterilized and if desired mixed with auxiliary agents, e.g., lubricants, preservatives, stabilizers, wetting agents, emulsifiers, salts for influencing osmotic pressure buffers, coloring, flavoring and/or aromatic substances.

The oral pharmaceutical compositions of the present invention can be in the form of tablets, coated tablets, liquids, drops, gelcaps, troches, lozenges, aqueous or oily suspensions, multiparticulate formulations including dispersable powders, granules, pellets, matrix spheroids, beads or coated inert beads, emulsions, hard or soft capsules or syrups or elixirs, microparticles (e.g., microcapsules and microspheres) as well as buccal tablets.

The oral compositions may be prepared according to methods known in the art and such compositions may contain one or more agents selected from the group consisting of inert, non-toxic pharmaceutically acceptable excipients which are suitable for the manufacture of tablets. Such excipients include, for example an inert diluent such as lactose; granulating and disintegrating agents such as cornstarch; binding agents such as starch; and lubricating agents such as magnesium stearate. The tablets may be uncoated or they may be coated by known techniques for elegance or to delay release of the active ingredients. Formulations for oral use may also be presented as hard gelatin capsules wherein the active ingredients is mixed with an inert diluent.

Aqueous suspensions preferably contain the opioid in a mixture that has one or more excipients suitable as suspending agents, for example, pharmaceutically acceptable synthetic gums such as hydroxypropylmethylcellulsoe or natural gums. Oily suspensions may be formulated by suspending the above-identified combination of drugs in a vegetable oil or mineral oil. The oily suspensions may contain thickening agent such as beeswax or cetyl alcohol. A syrup, or elixir, can be used, wherein a sweetened vehicle is employed.

The pharmaceutical oral compositions of the present invention comprise effective amounts of opioid antagonist and agonist (at last one of each) in a sustained release formulation. For example, a sustained release carrier can be included in the formulation to provide a release of the opioid antagonist over a 12 to 24 hour period. As used herein an effective amount of opioid means that the amount is sufficient to provide the desired therapeutic effect within the desired period of time. The therapeutic effect may also be the effect of an antagonist.

For example the sustained release oral dosage form which is effective for 24 hours at steady state conditions includes from about 1 to about 640 mg of oxycodone or a pharmaceutically acceptable salt thereof (e. g., oxycodone hydrochloride). Preferably the sustained release oral dosage form includes from about 5 to about 500 mg oxycodone or a pharmaceutically acceptable salt thereof, more preferably from about 10 to about 320 mg oxycodone or a pharmaceutically acceptable salt thereof and even more preferably from about 10 to about 160 mg oxycodone or a pharmaceutically acceptable salt thereof.

For example the sustained release oral dosage form which is effective for 12 hours at steady state conditions includes from about 1 to about 160 mg of oxycodone or a pharmaceutically acceptable salt thereof (e. g., oxycodone hydrochloride).

Other opioids may be present in amounts that are equivalent to the above mentioned oxycodone amounts with regard to the desired therapeutic effect.

In certain preferred embodiments, the oral dosage form includes a sustained-release material which is incorporated into a matrix along with the at least one opioid, to provide for the sustained release of the agent. The sustained-release material may be hydrophobic or hydrophilic as desired. The oral dosage form of the present invention may be prepared as granules, spheroids or matrix multiparticulates, which comprise the at least one opioid in a sustained release matrix which may be compressed into a tablet or encapsulated. The oral dosage form of the present invention may optionally include other pharmaceutically acceptable ingredients (e.g. diluents, binders, colorants, lubricants).

In certain other embodiments, the oral dosage form of the present invention may be an osmotic dosage form having a push or displacement composition as one of the layers of a bilayer core for pushing the at least one opioid from the dosage form, and a semipermeable wall composition surrounding the core, wherein the wall has at least one exit means or passageway for delivering the at least one opioid from the dosage form. Alternatively, the core of the osmotic dosage form may comprise a single layer core including a controlled release polymer and the at least one opioid.

Preferably the dosage forms of the present invention provides an effect for at least about 12 hours after administration.

### SUSTAINED-RELEASE MATRIX FORMULATIONS

In preferred embodiments of the present invention, the formulation can be a matrix with the at least one opioid interdispersed in the sustained release carrier, to provide for the sustained release of the at least one opioid.

A list of suitable sustained-release materials which may be included in a sustained-release matrix according to the invention include hydrophilic and/or hydrophobic materials, such as gums, cellulose ethers, acrylic resins, protein derived materials, waxes, shellac, and oils such as hydrogenated castor oil and hydrogenated vegetable oil. However, any pharmaceutically acceptable hydrophobic or hydrophilic sustained-release material which is capable of imparting sustained-release of the at least one opioid may be used in accordance with the present invention. Preferred sustained-release polymers include alkylcelluloses such as ethylcellulose, acrylic and methacrylic acid polymers and copolymers; and cellulose ethers, especially hydroyalkylcelluloses (especiall hydroxypropylmethylcellulose) and carboxyalkylcelluloses. Preferred acrylic and methacrylic acid polymers and copolymers include methyl methacrylate, methyl methacylate copolymers, ethoxyethyl methacrylates, ethyl acrylate, trimethyl ammonioethyl methacrylate, cyanoethyl methacrylate, aminoalkyl methacrylate copolymer, poly(acrylic acid), Poly(methacylic acid), methacrylic acid alkylamine copolymer, poly(methyl) methacrylate, poly(methacrylic acid)(anhydride), polymethacrylate, polyacrylamide, poly(methacrylic acid anhydride), and glycidyl methacrylate copolymers. Certain preferred embodiments utilise mixtures of any of the foregoing sustained-release materials in the matrix of the invention.

The matrix also may include a binder. In such embodiments, the binder preferably contributes to the sustained-release of the opoid from the sustained-release matrix.

If an additional hydrophobic binder material is included, it is preferably selected from natural and synthetic waxes, fatty acids, fatty alcohols, and mixtures of the same. Examples include beeswax, carnauba wax,stearic acid and stearyl alcohol. In certain preferred embodiments, a combination of two or more hydrophobic binder materials are included in the matrix formulations.

Preferred hydrophobic binder materials which may be used in accordance with the present invention include digestible, long chain (C₈-C₅₀, especially C₁₂-C₄₀), substituted or unsubstituted hydrocarbons, such as fatty acids, fatty alcohols, glyceryl esters of fatty acids, mineral and vegetable oils, natural and synthetic waxes and polyakylene glycols. Hydrocarbons having a melting point of between 25 ° and 90 °C are preferred. Of the long-chain hydrocarbon binder materials, fatty (aliphatic) alcohols are preferred in certain embodiments. The oral dosage form may contain up to 80 % (by weight) of at least one digestible, long chain hydrocarbon.

In certain embodiments, the hydrophobic binder material may comprise natural or synthetic waxes, fatty alcohols (such as lauryl, myristyl, stearyl, cetyl or preferably cetostearyl alcohol), fatty acids, including fatty acid esters, fatty acid glycerides (mono-, di-, and tri-glycerides), hydrogenated fats, hydrocarbons, normal waxes, stearic acid, stearyl alcohol and hydrophobic and hydrophilic materials having hydrocarbon backbones. Suitable waxes include, for example; beeswax, glycowax, castor wax and carnauba wax. For purposes of the present invention, a wax-like substance is defined as any material which is normally solid at room temperature and has a melting point of from about 30 to about 100 °C.

In certain preferred embodiments, the dosage form comprises a sustained release matrix comprising the at least one opioid and at least one water soluble hydroxyalkyl cellulose, at least one C₁₂-C₃₆, especially C₁₄-C₂₂, aliphatic alcohol and, optionally, at least one polyalkylene glycol. The hydroxyalkyl cellulose is preferably a hydroxy C₁-C₆ alkyl cellulose, such as hydroxypropoylcellulose, hydroxypropylmethylcellulose and, especially, hydroxethyl cellulose. The amount of the at least one hydroxyalkyl cellulose in the present oral dosage form may be determined, inter alia, by the precise rate of opioid release required.

In certain other preferred embodiments, the dosage form comprises a sustained release matrix comprising the at least one opioid and at least one acrylic resin, at least one C₁₂-C₃₆, especially C₁₄-C₂₂, aliphatic alcohol and, optionally, at least one polyalkylene glycol. The acrylic resin includes acrylic acid and methyacrylic acid copolymers, methyl methacrylate copolymers, ethoxyethyl methacrylates, cynaoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolamer, poly(methacrylic acid anhydride) and glycidyl methacrylate copolymers. In certain preferred embodiments, the acrylic polymer is comprised of one or more ammonio methacrylate copolymers. Ammonio methacrylate copolymers are well known in the art, and are described as fully polymerised copolymers of acrylic and methacrylic acid esters with a low content of quaternary ammonium groups. Preferably the acrylic resin is an acrylic polymer or an acrylic copolymer such as poly(meth)acrylate or methacrylic acid - ethyl acrylate copolymer or poly(meth)acrylate copolymerised with trimethyl ammonium (meth)acrylate chloride, such as poly(meth)acrylate with 5% trimethyl ammonium methacrylate chloride. The amount of the at least one acrylic resin in the present oral dosage form may be determined, inter alia, by the precise rate of opioid release required. In order to obtain a desirable dissolution profile, it may be necessary to incorporate two or more ammonio methacrylate copolymers having differing physical properties, such as different molar ratios of the quaternary ammonium groups to the neutral (meth)acrylic esters. Certain methacrylic acid ester-type polymers are useful for preparing pH-dependent matrices which may be used in accordance with the present invention. For example, there are a family of copolymers synthesized from diethylaminoethyl methacrylic acid copolymer or polymeric methacrylates, commercially available as Eudragit^{(R)} from Röhm Tech, Inc. There are several different types of Eudragit^{(R)}. For example, Eudragit E is an example of a methacrylic acid copolymer which does not swell at about pH <5.7 and is soluble at about pH >6. Eudragit S does not swell at about pH <6.5 and is soluble at about pH >7. Eudragit RL and Eudragit RS are water swellable, and the amount of water absorbed.by these polymers is pH-dependent, however, dosage forms with Eudragit RL and RS are pH-independent. In certain preferred embodiments, the acrylic matrix comprises a mixture of two acrylic resins commercially available from Rohm Pharma under the Tradenames Eudragit^{(R)} RL30D and Eudragit^{(R)} RS30D, respectively. Eudragit^{(R)} RL30D and Eudragit^{(R)} RS30D are copolymers of acrylic and methacrylic esters with a low content of quaternary ammonium groups, the molar ratio of ammonium groups to the remaining neutral (meth)acrylic esters being 1:20 in Eudragit^{(R)} RL30D and 1:40 in Eudragit^{(R)} RS30D. The mean molecular weight is about 150,000. The code designations RL (high permeability) and RS (low permeability) refer to the permeability properties of these agents. Eudragit^{(R)} RL/RS mixtures are insoluble in water and in digestive fluids. However, coatings formed from the same are swellable and permeable in aqueous solutions and digestive fluids. The Eudragit^{(R)} RL/RS dispersions of the present invention may be mixed together in any desired ration in order to ultimately obtain a sustained-release formulation having a desirable dissolution profile. Desirable sustained-release formulations may be obtained, for instance, from a retard matrices derived from Eudragit^{(R)} RL, Eudragit^{(R)} RL and Eudragit^{(R)} RS, and Eudragit^{(R)} RL and Eudragit^{(R)} RS. Of course, one skilled in the art will recognize that other acrylic polymers may also be used, such as, for example, Eudragit^{(R)} L.

The aliphatic alcohol may be, for example, lauryl alcohol, myristyl alcohol, cetostearyl alcohol or stearyl alcohol. In particularly preferred embodiments of the present oral dosage form, however, the at least one aliphatic alcohol is cetyl alcohol or cetostearyl alcohol. The amount of the aliphatic alcohol in the present oral dosage form may be determined, as above, by the precise rate of opioid release required. It may also depend on whether at least one polyalkylene glycol is present in or absent form the oral dosage form. In the absence of at least one polyalkylene glycol, the oral dosage form preferably contains between about 20 % and about 50 % (by wt) of the aliphatic alcohol. When a polyalkylene glycol is present in the oral dosage form, then the combined weight of the aliphatic alcohol and the polyalkylene glycol preferably constitutes between about 20 % and about 50 % (by wt) of the total dosage form.

In one preferred embodiment, the ration of, e.g., the at least one hydroxyalkyl cellulose or acrylic resin to the at least one aliphatic alcohol/polyalkylene glycol determines, to a considerable extent, the release rate of the opoid from the formulation. In certain embodiments, a ration of the hydroxyalkyl cellulose to the aliphatic alcohol/polyalkylene glycol of between 1:1 and 1:4 is preferred, with a ratio of between 1:2 and 1:3 being particularly preferred.

In certain embodiments, the polyalkylene glycol may be, for example, polypropylene glycol, or polyethylene glycol which is preferred. The average molecular weight of the at least one polyallylene glycol is preferably between 1,000 and 15,000, especially between 1,500 and 12,000.

Another suitable sustained-release matrix comprises an alkylcellulose (especially ethylcellulose), a C₁₂ to C₃₆ aliphatic alcohol and, optionally, a polyalkylene glycol.

In addition to the above ingredients, a sustained-release matrix may also contain suitable quantities of other materials, e.g., diluents, lubricants, binders, granulating aids, colorants, flavorants and glidants that are conventional in the pharmaceutical art.

In order to facilitate the preparation of a solid, sustained-release oral dosage form according to this invention incorporation the opioid in the matrix may be effected, for example, by:
(a) forming granules comprising at least one hydrophobic and/or hydrophilic material as set forth above (e.g., a water soluble hydroxyalkyl cellulose or an acrylic resin) together with the opioid;
(b) mixing the granules containing at lest one hydrophobic and/or hydrophilic material with at least one C₁₂-C₃₆ aliphatic alcohol, (and, in case, other matrix components) and
(c) optionally, compressing and shaping the granules.

The granules may be formed by any of the procedures well-known to those skilled in the art of pharmaceutical formulation. For example, in one preferred method, the granules may be formed by wet granulating hydroxyalkyl cellulose/opoid with water. In a particularly preferred embodiment of this process, the amount of water added during the wet granulation step is preferably between 1.5 and 5 times, especially between 1.75 and 3.5 times, the dry weight of opioid.

A sustained-release matrix can also be prepared by, e.g., melt-granulation or melt-extrusion techniques. Generally, melt-granulation techniques involve melting a normally solid hydrophobic binder material, e.g., a wax, and incorporating a powdered drug therein. To obtain a sustained release dosage form, it may be necessary to incorporate a hydrophobic sustained-release material, e.g. ethylcellulose or a water-insoluble acrylic polymer, into the molten wax hydrophobic binder material. Examples of sustained-release formulations prepared via melt-granulation techniques are found, e.g., in U.S. Patent No. 4,861,598.

The additional hydrophobic binder material may comprise one or more water-insoluble wax-like thermoplastic substances possibly mixed with one or more wax-like thermoplastic substances being less hydrohphobic than said one or more water-insoluble wax-like substances in the formulation should be substantially nondegradable and insoluble in gastrointestinal fluids during the initial release phases. Useful water-insoluble wax-like binder substances may be those with a water-solubility that is lower than about 1:5,000 (w/w).

Extruded formulations employing starch, as e.g. disclosed in DE 19918325 A1, can be advantageously employed in the context of the invention.

The preparation of a suitable melt-extruded matrix according to the present invention may, for example, include the steps of blending the least one opioid, together with a sustained release material and preferably a binder material to obtain a homogenous mixture. The homogenous mixture is then heated to a temperature sufficient to at least soften the mixture sufficiently to extrude the same. The resulting homogenous mixture is then extruded, e.g., using a twin-screw extruder, to form strands. The extrudate is preferably cooled and cut into multiparticulates by any means known in the art. The matrix muliparticulates are then divided into unit doses. The extrudate preferably has a diameter of from about 0.1 to about 5 mm and provides sustained release of the at least one opioid for a time period of at least about 24 hours.

An optional process for preparing the melt extruded formulations of the present invention includes directly metering into an extruder a hydrophobic sustained release material, the at least one opioid and an optional binder material; heating the homogenous mixture; extruding the homogenous mixture to thereby form stands; cooling the strands containing the homogeneous mixture; cutting the strands into matrix multiparticulates having a size from about 0.1 mm to about 12 mm; and dividing said particles into unit doses. In this aspect of the invention a relatively continuous manufacturing procedure is realized.

Plasticizers, such as those described above, may be included in melt-extruded matrices. The plasticizer is preferably included as from about 0.1 to about 30 % by weight of the matrix. Other pharmaceutical excipients, e.g., talc, mono or poly saccharides, colorants, flavorants, and lubricants may be included in the sustained release matrices of the present invention as desired. The amounts included will depend upon the desired characteristic to be achieved.

The diameter of the extruder aperture or exit port can be adjusted to vary the thickness of the extruded strands. Furthermore, the exit part of the extruder need not be round; it can be oblong or rectangular. The exiting strands can be reduced to particles using a hot wire cutter or a guillotine.

A melt extruded matrix multiparticulate system can be, for example, in the form of granules, spheroids or pellets depending upon the extruder exit orifice. For purposes of the present invention, the terms "melt-extruded matrix multiparticulate(s)" and "melt-extruded matrix multiparticulate system(s)" and "melt-extruded matrix particles" shall refer to a plurality of units, preferably within a range of similar size and/or shape and containing one or more actives and one or more excipients, preferably including a hydrophobic sustained release material as described herein. Preferably the melt-extruded matrix multiparticulates will be of a range of from about 0.1 to about 12 mm in length and have a diameter of from about 0.1 to about 5 mm. In addition, it is to be understood that the melt-extruded matrix multiparticulates can be any geometrical shape within this size range. In certain embodiments, the extrudate may simply be cut into desired lengths and divided into unit doses of the therapeutically active agent without the need of a spheronization step.

In one preferred embodiment, oral dosage forms are prepared that include an effective amount of melt-extruded matrix multiparticulates within a capsule. For example, a plurality of the melt-extruded matrix multiparticulates may be placed in a gelatin capsule in an amount sufficient to provide an effective sustained release dose when ingested and contacted by gastrointestinal fluid.

In another embodiment, a suitable amount of the multiparticulate extrudate is compressed into an oral tablet using conventional tableting equipment using standard techniques. Techniques and compositions for making tablets (compressed and molded), capsules (hard and soft gelatin) and pills are also described in Remington's Pharmaceutical Sciences, (Arthur Oso, editor), 1553-1593 (1980)

In yet another preferred embodiment, the extrudate can be shaped into tablets as set forth in U. S. Patent No. 4,957,681 (Klimesch, et. al.).

Optionally, the sustained-release matrix multiparticulate systems, tablets, or capsules can be coated with a sustained release coating such as the sustained release coatings described herein. Such coatings preferably include a sufficient amount of hydrophobic and/or hydrophilic sustained-release material to obtain a weight gain level from about 2 to about 25 percent, although the overcoat may be greater depending upon, e.g., the desired release rate.

The dosage forms of the present invention may further include combinations of melt-extruded matrix multiparticulates containing the at least one opioid. Furthermore, the dosage forms can also include an amount of an immediate release therapeutically active opioid for prompt therapeutic effect. The immediate release opioid may be incorporated, e.g., as separate multiparticulates within a gelatin capsule, or may be coated on the surface of, e.g., melt-extruded matrix multiparticulates.

The sustained-release profile of the melt-extruded formulations of the invention can be altered, for example, by varying the amount of sustained-release material, by varying the amount of plasticizer relative to other matrix constituents, by varying the amount of hydrophobic material, by the inclusion of additional ingredients or excipients or by altering the method of manufacture.

In other embodiments of the invention, melt-extruded formulations are prepared without the inclusion of the at least one opioid, which is added thereafter to the extrudate. Such formulations typically will have the opioid blended together with the extruded matrix material, and then the mixture would be tableted in order to provide a slow release formulation. Such formulations may be advantageous, for example, when the therapeutically active agent included in the formulations is sensitive to temperatures needed for softening the hydrophobic material and/or the retardant material.

Typical melt-extrusion production systems suitable for use in accordance with the present invention include a suitable extruder drive motor having variable speed and constant torque control, start-stop controls, and a meter. In addition, the production system will include a temperature control console which includes temperature sensors, cooling means and temperature indicators throughout the length of the extruder. In addition, the production system will include an extruder such as a twin-screw extruder which consists of two counter-rotating intermeshing screws enclosed within a cylinder of barrel having an aperture or die at the exit thereof. The feed materials enter through a feed hopper and are moved through the barrel by the screws and are forced through the die into strands which are thereafter conveyed such as by a continuous movable belt to allow for cooling and being directed to a pelletizier or other suitable device to render the extruded ropes into the matrix multiparticulate system. The pelletizer can consist of rollers, fixed knife and rotating cutter. Suitable instruments and systems are available from distributors such as C.W. Brabender Instruments, Inc. of South Hackensack, New Jersey. Other suitable apparatus will be apparent to those of ordinary skill in the art.

In the preparation of melt-extruded matrix multiparticulates as set forth above the amount of air included in the extrudate can be controlled and the release rate of the at least one opioid thereof may be altered.

Thus the melt-extruded product is prepared in a manner which substantially excludes air during the extrusion phase of the process. This may be accomplished, for example, by using a Leistritz extruder having a vacuum attachment. The extruded matrix multiparticulates prepared according to the invention using a Leistritz extruder under vacuum provides a melt-extruded product having different physical characteristics. In particular, the extrudate is substantially non-porous when magnified, e.g., using a scanning electron microscope. Such substantially non-porous formulations may provide a faster release of the therapeutically active agent, relative to the same formulation prepared without vacuum. Scanning electron micrographs of the matrix multiparticulates prepared using an extruder under vacuum appear very smooth, as compared to multiparticulates prepared without vacuum. It has been observed that in at least certain formulations, the use of extrusion under vacuum provides an extruded matrix multiparticulates product which is more pH-dependent than its counterpart formulation prepared without vacuum.

Alternatively, the melt-extruded product is prepared using a Werner-Pfleiderer twin screw extruder.

In certain embodiments, a spheronizing agent is added to a granulate or matrix multiparticulate and then spheronized to produce sustained release spheroids. The spheroids are then optionally overcoated with a sustained release coating by methods such as those described above.

Spheronizing agents which may be used to prepare the matrix multiparticulate formulations of the present invention include any art-known spheronizing agent.

Cellulose derivatives are preferred, and microcrystalline cellulose is especially preferred. A suitable microcrystalline cellulose is, for example, the material sold as Avicel PH101 (TradeMark, FMC Corporation). The spheronizing agent is preferably included as about 1 to about 99 % of the matrix multiparticulate by weight.

In certain embodiments, in addition to the active ingredient and spheronizing agent, the spheroids may also contain a binder. Suitable binders, such as low viscosity, water soluble polymers, will be well known to those skilled in the pharmaceutical art. However, water soluble hydroxy lower alkyl cellulose, such as hydroxy propyl cellulose, are preferred. Additionally (or alternatively) the spheroids may contain a water insoluble polymer, especially an acrylic polymer, an acrylic copolymer, such as a methacrylic acid-ethyl acrylate copolymer, or ethyl cellulose.

In certain embodiments, a sustained release coating is applied to the sustained release spheroids, granules, or matrix multiparticulates. In such embodiments, the sustained-release coating may include a water insoluble material such as (a) a wax, either alone or in admixture with a fatty alcohol; or (b) shellac or zein, the coating is preferably derived from an aqueous dispersion of the hydrophobic sustained release material.

In certain embodiments, it is necessary to overcoat the sustained release spheroids, granules, or matrix multiparticulates comprising the at least one opioid and sustained release carrier with a sufficient amount of the aqueous dispersion of, e.g., alkylcellulose or acrylic polymer, to obtain a weight gain level form about 2 to about 50 %, e.g., about 2 to about 25 %, in order to obtain a sustained-release formulation. The overcoat may be lesser or greater depending upon, e.g., the desired release rate, the inclusion of plasticizer in the aqueous dispersion and the manner of incorporation of the same. Cellulosic materials and polymers, including alkylcelluloses, are sustained release materials well suited for coating the sustained release spheroids, granules, or matrix multiparticulates according to the invention. Simply by way of example, one preferred alkylcellulosic polymer is ethylcellulose, although the artisan will appreciate that other cellulose and/or alkylcellulose polymers may be readily employed, singly or in any combination, as all or part of a hydrophobic coating according to the invention.

One commercially-available aqueous dispersion of ethylcellulose is Aquacoat^{(R)} (FMC Corp., Philadelphia, Pennsylvania, U.S.A.). Aquacoat^{(R)} is prepared by dissolving the ethylcellulose in a water-immiscible organic solvent and then emulsifying the same in water in the presence of a surfactant and a stabilizer. After homogenization to generate submicron droplets, the organic solvent is evaporated under vacuum to form a pseudolatex. The plasticizer is not incorporated in the pseudolatex during the manufacturing phase. Thus, prior to using the same as a coating, it is necessary to intimately mix the Aquacoat^{(R)} with a suitable plasticizer prior to use.

Another aqueous dispersion of ethylcellulose is commercially available as Surelease^{(R)} (Colorcon, Inc., Weset Point, Pennsylvania, U.S.A.). This product is prepared by incorporating plasticizer into the dispersion during the manufacturing process. A hot melt of a polymer, plasticizer (dibutyl sebacate), and stabilizer (oleic acid) is prepared as a homogeneous mixture, which is then diluted with an alkaline solution to obtain an aqueous dispersion which can be applied directly to the sustained release spheroids, granules, or matrix multiparticulates.

In other preferred embodiments of the present invention, the sustained release material comprising the sustained-release coating is a pharmaceutically acceptable acrylic polymer, including acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate)), polymethacrylate, poly(methyl methacrylate) copolymer, polyacrylamide, aminoalkyl methacrylate copolymer, poly(methacrylic acid anhydride), and glycidyl methacrylate copolymers. Useful acrylic polymers are the resins known under the Tradename Eudragit^{(R)}, commercially available from Rohm Pharma. These acrylic resins can be tailored to provide a pH dependent or a pH independent release rate of the active.

In addition to the above ingredients, the spheroids, granules or matrix multiparticulates may also contain suitable quantities of other materials, e.g., diluents, lubricants, binders, granulating aids, colorants, flavorants and glidants that are conventional in the pharmaceutical art in amounts up to about 50 % by weight of the formulation if desired. The quantities of these additional materials will be sufficient to provide the desired effect to the desired formulation.

Specific examples of orally acceptable carriers and excipients that may be used to formulate oral dosage forms are described in the Handbook of Pharmaceutical Excipients, American Pharmaceutical Association (1986).

It has further been found that the addition of a small amount of talc to the sustained release coating reduces the tendency of the aqueous dispersion to stick during processing, and acts as a polishing agent.

If oxycodone is used for the preparation the formulation is chosen the matrix comprises at least one acrylic resin and at least one C₁₂-C₃₆ aliphatic alcohol as they are described above. The preparation is preferably accomplished by the above described granulation method with the above described preferred amounts of ingredients.

If oxycodone and naloxone are used for a combination preparation the formulation is chosen to ensure that the active compounds are released from the preparation in a sustained, independent and invariant manner. Preferably those formulations are storage stable.

The terms " released from the preparation in a sustained, independent and invariant manner" and "storage stable" as used herein are defined as in PCT/EP 03/03541.

If oxycodone and naloxone are used for a combination preparation the formulation is chosen that it comprises a release matrix that has the character of a substantially non-water- or non-buffer-swellable and non-erosive diffusion matrix as defined in PCT/EP 03/0354.

If oxycodone and naloxone are used for a combination preparation a formulation is especially preferred that comprises ethylcellulose or Surelease® E-7-7050 as a matrix-building substance, stearyl alcohol as fatty alcohol, magnesium stearate as lubricant, lactose as filler and povidone as a granulating aid.

Such preparations can be produced as all common application forms which, on principle, are suitable for retardation formulations and which ensure that the active compounds are released in a manner as outlined above. Especially suitable are tablets, multi-layer tablets and capsules. Additional application forms like granules or powders can be used, with only those applications forms being admissible that provide a sufficient retardation and a release behaviour as outlined above.

Such pharmaceutical preparations may also comprise film coatings. However, it has to be ensured that the film coatings do not negatively influence the release properties of the active compounds from the matrix and the storage stability of the active compounds within the matrix. Such film coatings may be colored or may comprise a initial dosage of the active compounds if required. The active compounds of this initial dosage will be immediately released so that the therapeutically effective blood plasma level is reached very quickly.

A detailed description of the preparation of these oxycodone/naloxone combination preparations can be taken from PCT/EP 03/03541.

### Process for preparing matrix beads

Sustained-release dosage forms according to the present invention may also be prepared as matrix beads formulations. The matrix beads include a spheronising agent and the at least one opioid.

Opioids preferably comprise from about 0.01 to about 99 % by weight of the matrix bead by weight. It is preferable that the opioids are included as about 0.1 to about 50 % by weight of the matrix bead.

Spheronising agents which may be used to prepare the matrix bead formulations of the present invention include any art-known spheronising agent. Cellulose derivatives are preferred, and microcrystalline cellulose is especially preferred. A suitable microcrystalline cellulose is, for example, the material sold as Avicel PH 101 (TradeMark, FMC Corporation). The spheronising agent is preferably included as about 1 to about 99 % of the matrix bead by weight.

In addition to the active ingredient and spheronizing agent, the spheroids may also contain a binder. Suitable binders, such as low viscosity, water soluble polymers, will be well known to those skilled in the pharmaceutical art. However, water soluble hydroxy lower alkylcellulose, such as hydroxypropylcellulose, are preferred.

In addition to the opioids and spheronising agent, the matrix bead formulations of the present invention may include a controlled release material such as those described hereinabove. Preferred controlled-release materials for inclusion in the matrix bead formulations include acrylic and methacrylic acid polymers or copolymers, and ethylcellulose. When present in the formulation, the controlled-release material will be included in amounts of from about 1 to about 80 % of the matrix bead, by weight. The controlled-release material is preferably included in the matrix bead formulation in an amount effective to provide controlled release of the opioids from the bead.

Pharmaceutical processing aids such as binders, and diluents may be included in the matrix bead formulations. Amounts of these agents included in the formulations will vary with the desired effect to be exhibited by the formulation.

The matrix beads may be overcoated with a controlled-release coating including a controlled-release material such as those described hereinabove. The controlled-release coating can be applied to a weight gain of from about 5 to about 30 %. The amount of the controlled release coating to be applied will vary according to a variety of factors, e.g., the composition of the matrix beads.

Matrix beads are generally prepared by granulating the spheronizing agent together with the agent, e.g. by wet granulation. The granulate is then spheronized to produce the matrix beads. The matrix beads are then optionally overcoated with the controlled release coating by methods such as those described hereinabove.

Another method for preparing matrix beads, for example, by (a) forming granules comprising at least one water soluble hydroxyalkyl cellulose and an opioid (b) mixing the hydroxyalkyl cellulose containing granules with at least one C₁₂ - C₃₆ aliphatic alcohol; and (c) optionally, compressing and shaping the granules. Preferably, the granules are formed by wet granulating the hydroxyalkyl cellulose/opioid with water.

In yet another alternative embodiment, spheronizing agent, together with the active ingredient can be spheronized to form spheroids. Microcrystalline cellulose is preferred. A suitable microcrystalline cellulose is, for example, the material sold as Avicel PH 101 (Trade mark, FMC Corporation). In such embodiments, in addition to the active ingredient and spheronizing agent, the spheroids may also contain a binder. Suitable binders, such as low viscosity, water soluble polymers, will be well known to those skilled in the pharmaceutical art. However, water soluble hydroxy lower alkyl cellulose, such as hydroxy propyl cellulose, are preferred. Additionally (or alternatively) the spheroids may contain a water insoluble polymer, especially an acrylic polymer, an acrylic copolymer, such as a methacrylic acid-ethyl acrylate copolymer, or ethyl cellulose. In such embodiments, the sustained-release coating will generally include a water insoluble material such as (a) a wax, either alone or in admixture with a fatty alcohol; or (b) shellac or zein.

In one especially preferred embodiment, the oral dosage form comprises an effective number of controlled release spheroids contained within a gelatin capsule.

In another preferred embodiment of the present invention, the sustained-release dosage form comprises spheroids containing the active ingredient coated with a controlled-release coating including a controlled release material. The term spheroid is known in the pharmaceutical art and means, e.g., a spherical granule having a diameter of between 0.1 mm and 2.5 mm, or between 0.5 mm and 2 mm. The diameter can be higher or lower than disclosed above.

The spheroids are preferably film coated with a controlled release material that permits release of the opioid at a controlled rate in an aqueous medium. The film coat is chosen so as to achieve, in combination with the other stated properties, the desired in-vitro release rates The sustained-release coating formulations of the present invention preferably produce a strong, continuous film that is smooth and elegant, capable of supporting pigments and other coating additives, non-toxic, inert, and tack-free.

### SUSTAINED-RELEASE COATING FORMULATIONS

The oral dosage forms of the present invention may optionally be coated with one or more coatings suitable for the regulation of release of for the protection of the formulation. In one embodiment, coatings are provided to permit either pH-dependent or pH-independent release, e.g., when exposed to gastrointestinal fluid. When a pH-independent coating is desired, the coating is designed to achieve optimal release regardless of pH-changes in the environmental fluid, e.g., the GI tract, to avoid dose dumping. Other preferred embodiments include a pH-dependent coating that releases the opioid antagonist in desired areas of the gastro-intestinal (GI) tract, e.g., the stomach or small intestine. It is also possible to formulate compositions which release a portion of the dose in one desired area of the GI tract, e.g., the stomach, and release the remainder of the dose in another area of the GI tract, e.g., the small intestine.

Formulations according to the invention that utilize pH-dependent coatings may also impart a repeat-action effect whereby unprotected drug is coated over an enteric coat and is released in the stomach, while the remainder, being protected by the enteric coating, is released further down the gastrointestinal tract. Coatings which are pH-dependent may be used in accordance with the present invention include a controlled release material such as, e.g., shellac, cellulose acetate phthalate (CAP), polyvinyl acetate phthalate (PVAP), hydroxypropyl methylcellulose phthalate, and methacrylic acid ester copolymers and zein.

In another preferred embodiment, the present invention is related to a stabilized solid sustained dosage form comprising the opioid coated with a hydrophobic controlled release material selected from (i) an alkylcellulose; (ii) an acrylic polymer; or (iii) mixtures thereof The coating may be applied in the form of an organic or aqueous solution or dispersion.

In certain preferred embodiments, the controlled release coating is derived from an aqueous dispersion of the hydrophobic controlled release material. The coated substrate containing the opioid (e.g., a tablet core or inert pharmaceutical beads or spheroids) is then cured until an endpoint is reached at which the substrate provides a stable dissolution. The curing endpoint may be determined by comparing the dissolution profile (curve) of the dosage form immediately after curing to the dissolution profile (curve) of the dosage form after exposure to accelerated storage conditions of, e.g., at least one month at a temperature of 40 °C and a relative humidity of 75 %. These formulations are described in detail in U.S. Patent Nos. 5,273,760 and 5,286,493. Other examples of sustained-release release formulations and coatings which may be used in accordance with the present invention include Assignee's U.S. Patent Nos. 5,324,351, 5,356,467, and 5,472,712.

In preferred embodiments, the controlled release coatings include a plasticizer such as those described herein below.

In certain embodiments, it is necessary to overcoat the substrate comprising the opioid with a sufficient amount of the aqueous dispersion of e.g., alkylcellulose or acrylic polymer, to obtain a weight gain level from about 2 to about 50 %, e.g., about 2 to about 25 % in order to obtain a sustained-release formulation. The overcoat may be lesser or greater depending upon the physical properties of the therapeutically active agent and the desired release rate, the inclusion of plasticizer in the aqueous dispersions and the manner of incorporation of the same, for example.

### Alkylcellulose Polymers

Cellulosic materials and polymers, including alkylcelluloses are controlled release materials well suited for coating the substrates, e.g., beads or tablets according to the invention. Simply by way of example, one preferred alkylcellulosic polymer is ethylcellulose, although the artisan will appreciate that other cellulose and/or part of a hydrophobic coatings according to the invention.

One commercially available aqueous dispersion of ethylcellulose is Aquacoat^{(R)} (FMC Corp., Philadelphia, Pennsylvania, U.S.A.). Aquacoat^{(R)} is prepared by dissolving the ethylcellulose in a water-immiscible organic solvent and then emulsifying the same in water in the presence of a surfactant and a stabilizer. After homogenisation to generate submicron droplets, the organic solvent is evaporated under vacuum to form a pseudolatex. The plasticizer is not incorporated in the pseudolatex during the manufacturing phase. Thus, prior to using the same as a coating, it is necessary to intimately mix the Aquacoat^{(R)} with a suitable plasticizer prior to use.

Another aqueous dispersion of ethylcellulose is commercially available as Surelease^{(R)} (Colorcon, Inc., West Point, Pennsylvania, U.S.A.). This product is prepared by incorporating plasticizer into the dispersion during the manufacturing process. A hot melt of a polymer, plasticizer (dibutyl sebacate), and stabilizer (oleic acid) is prepared as a homogeneous mixture, which is then diluted with an alkaline solution to obtain an aqueous dispersion which can be applied directly onto substrates.

### Acrylic Polymers

In other preferred embodiments of the present invention, the controlled release material comprising the controlled-release coating is a pharmaceutically acceptable acrylic polymer, including acrylic acid and methyacrylic acid copolymers, methyl methacrylate copolymers, ethoxyethyl methacrylates, cynaoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolamer, poly(methacrylic acid anhydride) and glycidyl methacrylate copolymers.

In certain preferred embodiments, the acrylic polymer is comprised of one or more ammonio methacrylate copolymers. Ammonio methacrylate copolymers are well known in the art, and are described as fully polymerised copolymers of acrylic and methacrylic acid esters with a low content of quaternary ammonium groups.

In order to obtain a desirable dissolution profile, it may be necessary to incorporate two or more ammonio methacrylate copolymers having differing physical properties, such as different molar ratios of the quaternary ammonium groups to the neutral (meth)acrylic esters.

Certain methacrylic acid ester-type polymers are useful for preparing pH-dependent coatings which may be used in accordance with the present invention. For example, there are a family of copolymers synthesized from diethylaminoethyl methacrylic acid copolymer or polymeric methacrylates, commercially available as Eudragit^{(R)} from Röhm Tech, Inc. There are several different types of Eudragit^{(R)}. For example, Eudragit E is an example of a methacrylic acid copolymer which does not swell at aobut pH <5.7 and is soluble at about pH >6. Eudragit S does not swell at about pH <6.5 and is soluble at about pH >7. Eudragit RL and Eudragit RS are water swellable, and the amount of water absorbed by these polymers is pH-dependent, however, dosage forms coated with Eudragit RL and RS are pH-independent.

In certain preferred embodiments, the acrylic coating comprises a mixture of two acrylic resin lacquers commercially available from Rohm Pharma under the Tradenames Eudragit^{(R)} RL30D and Eudragit^{(R)} RS30D, respectively. Eudragit^{(R)} RL30D and Eudragit^{(R)} RS30D are copolymers of acrylic and methacrylic esters with a low content of quaternary ammonium groups, the molar ratio of ammonium groups to the remaining neutral (meth)acrylic esters being 1:20 in Eudragit^{(R)} RL30D and 1:40 in Eudragit^{(R)} RS30D. The mean molecular weight is about 150,000. The code designations RL (high permeability) and RS (low permeability) refer to the permeability properties of these agents. Eudragit^{(R)} RL/RS mixtures are insoluble in water and in digestive fluids. However, coatings formed from the same are swellable and permeable in aqueous solutions and digestive fluids.

The Eudragit^{(R)} RL/RS dispersions of the present invention may be mixed together in any desired ration in order to ultimately obtain a sustained-release formulation having a desirable dissolution profile. Desirable sustained-release formulations may be obtained, for instance, from a retardant coating derived from 100 % Eudragit^{(R)} RL, 50 % Eudragit^{(R)} RL and 50 % Eudragit^{(R)} RS, and 10 % Eudragit^{(R)} RL: Eudragit^{(R)} RS. Of course, one skilled in the art will recognize that other acrylic polymers may also be used, such as, for example, Eudragit^{(R)} L.

### Plasticizers

In embodiments of the present invention where the coating comprises an aqueous dispersion of a hydrophobic controlled release material, the inclusion of an effective amount of a plasticizer in the aqueous dispersion of hydrophobic material will further improve the physical properties of the controlled-release coating. For example, because ethylcellulose has a relatively high glass transition temperature and does not form flexible films under normal coating conditions, it is preferable to incorporate a plasticizer into an ethylcellulose coating containing controlled-release coating before using the same as a coating material. Generally, the amount of plasticizer included in a coating solution is based on the concentration of the film-former, e.g., most often from about 1 to about 50 percent by weight of the film-former. Concentration of the plasticizer, however, can only be properly determined after careful experimentation with the particular coating solution and method of application.

Examples of suitable plasticizers for ethylcellulose include water insoluble plasticizers such as dibutyl sebacate, diethyl phthalate, triethyl citrate, tibutyl citrate, and triacetin, although it is possible that other water-insoluble plasticizers (such as acetylated monoglycerides, phthalate esters or castor oil) may be used. Triethyl citrate is an especially preferred plasticizer for the aqueous dispersions of ethyl cellulose of the present invention.

Examples of suitable plasticizers for the acrylic polymers of the present invention include citric acid esters such as triethyl citrate NF XVI, tributyl citrate, dibutyl phthalate, and possibly 1,2-propylene glycol. Other plasticizers which have proved to be suitable for enhancing the elasticity of the films formed from acrylic films such as Eudragit^{(R)} RL/RS lacquer solutions include polyethylene glycols, propylene glycol, diethyl phthalate, castor oil, and triacetin. Triethyl citrate is an especially preferred plasticizer for the aqueous dispersions of ethyl cellulose of the present invention.

It has further been found that the addition of a small amount of talc to the controlled release coating reduces the tendency of the aqueous dispersion to stick during processing, and act as a polishing agent.

### Preparation of coated bead formulations

When an aqueous dispersion of hydrophobic material is used to coat substrates, e.g., inert pharmaceutical beads such as nu pariel 18/20 beads, a plurality of the resultant stabilized solid controlled-release beads may thereafter be places in a gelatin capsule in an amount sufficient to provide an effective controlled-release dose when ingested and contacted by an environmental fluid, e.g., gastric fluid or dissolution media.

The stabilized sustained-release bead formulations of the present invention slowly release the opioid antagonist, e.g., when ingested and exposed to gastric fluids, and then to intestinal fluids. The sustained-release profile of the formulations of the invention can be altered, for example, by varying the amount of overcoating with the aqueous dispersion of hydrophobic controlled release material, altering the manner in which the plasticizer is added to the aqueous dispersion of hydrophobic controlled release material, by varying the amount of plasticizer relative to hydrophobic controlled release material, by the inclusion of additional ingredients or excipients or by altering the method of manufacture. The dissolution profile of the ultimate product may also be modified, for example, by increasing or decreasing the thickness of the controlled release coating.

Substrates coated with a therapeutically active agent are prepared, e.g. by dissolving the therapeutically active agent in water and then spraying the solution onto a substrate, for example, nu pariel 18/20 beads, using a Wuster insert. Optionally, additional ingredients are also added prior to coating the beads in order to assist the binding of the opioid to the beads, and/or to color the solution. For example, a product which includes hydroxypropyl methylcellulose with or without colorant (e.g., Opadry^{(R)}, commercially available from Colorcon, Inc.) may be added to the solution and the solution mixed (e.g., for about 1 hour) prior to application of the same onto the substrate. The resultant coated substrate may then be optionally overcoated with a barrier agent, to separate the therapeutically active agent from the hydrophobic controlled-release coating.

An example of a suitable barrier agent is one which comprises hydroxypropyl methylcellulose. However, any film-former known in the art may be used. It is preferred that the barrier agent does not affect the dissolution rate of the final product.

The substrates may then be overcoated with an aqueous dispersion of the hydrophobic controlled release material. The aqueous dispersion of hydrophobic controlled release material preferably further includes an effective amount of plasticizer, e.g. triethyl citrate. Pre-formulated aqueous dispersions of ethylcellulose, such as Aquacoat^{(R)} or Surelease^{(R)}, may be used. If Surelease^{(R)} is used, it is not necessary to separately add a plasticizer. Alternatively, pre-formulated aqueous dispersions of acrylic polymers such as Eudragit^{(R)} can be used.

The coating solutions of the present invention preferably contain, in addition to the film-former, plasticizer, and solvent system (i.e., water) a colorant to provide elegance and product distinction. Color may be added to the solution of the therapeutically active agent instead, or in addition to the aqueous dispersion of hydrophobic material. For example, color can be added to Aquacoat^{(R)} via the use of alcohol or propylene glycol based color dispersions, milled aluminium lakes and opacifiers such as titanium dioxide by adding color with shear to water soluble polymer solution and then using low shear to the plasticized Aquacoat^{(R)}. Alternatively, any suitable method of providing color to the formulations of the present invention may be used. Suitable ingredients for providing color to the formulation when an aqueous dispersion of an acrylic polymer is used include titanium dioxide and color pigments, such as iron oxide pigments. The incorporation of pigments, may, however, increase the retard effect of the coating.

The plasticized aqueous dispersion ofhydrophobic controlled release material may be applied onto the substrate comprising the therapeutically active agent by spraying using any suitable spray equipment known in the art. In a preferred method, a Wurster fluidized-bed system is used in which an air jet, injected from underneath fluidizes the core material and effects drying while the acrylic polymer coating is sprayed on. A sufficient amount of the aqueous dispersion of hydrophobic material to obtain a predetermined controlled-release of said therapeutically active agent when said coated substrate is exposed to aqueous solutions, e.g. gastric fluid, is preferably applied, taking into account the physical characteristics of the therapeutically active agent or the manner of incorporation of the plasticizer. After coating with the hydrophobic controlled release material, a further overcoat of a film-former, such as Opadry^{(R)}, is optionally applied to the beads. This overcoat is provided, if at all, in order to substantially reduce agglomeration of the beads.

The release of the therapeutically active agent from the sustained-release formulation of the present invention can be further influenced, i.e., adjusted to a desired rate, by the addition of one or more release-modifying agents, or by providing one or more passageways through the coating. The ratio of hydrophobic controlled release material to water soluble material is determined by, among other factors, the release rate required and the solubility characteristics of the materials selected.

The release-modifying agents which function as pore-formers may be organic or inorganic, and include materials that can be dissolved, extracted or leached form the coating in the environment of use. The pore-formers may comprise one or more hydrophilic materials such as hydroxypropylmethylcellulose.

The controlled-release coatings of the present invention can also include erosion-promoting agents such as starch and gums.

The controlled-release coatings of the present invention can also include materials useful for making microporous lamina in the environment of use, such as polycarbonates comprised of linear polyesters of carbonic acid in which carbonate groups reoccur in the polymer chain.

The release-modifying agent may also comprise a semi-permeable polymer. In certain preferred embodiments, the release-modifying agent is selected from hydroxypropylmethylcellulose, lactose, metal stearates, and mixtures of any of the foregoing.

The controlled-release coatings of the present invention may also include an exit means comprising at least one passageway or orifice. The passageway may be formed by such methods as those disclosed in U.S. Patent Nos. 3,845,770; 3,916,889; 4,063,064; and 4,088,864. The passageway can have any shape such as round, triangular, square, elliptical or irregular.

Another method of producing sustained release release bead formulations suitable for about 24-hour administration is via powder layering. U.S. Patent No. 5,411,745 teaches preparation of 24-hour morphine formulations prepared via powder layering techniques utilizing a processing aid consisting essentially of hydrous lactose impalpable. The powder-layered beads are prepared by spraying an aqueous binder solution onto inert beads to provide a tacky surface, and subsequently spraying a powder that is a homogenous mixture of morphine sulfate and hydrous lactose impalpable onto the tacky beads. The beads are then dried and coated with a hydrophobic material such as those described hereinabove to obtain the desired release of drug when the final formulation is exposed to environmental fluids. An appropriate amount of the controlled release beads are then, e.g. encapsulated to provide a final dosage form which provides effective plasma concentrations of morphine for about 24 hours.

### SUSTAINED RELEASE OSMOTIC DOSAGE

Sustained release dosage forms according to the present invention may also be prepared as osmotic dosage formulations. The osmotic dosage forms preferably include a bilayer core comprising a drug layer and a delivery or push layer, wherein the bilayer core is surrounded by a semipermeable wall and optionally having at least one passageway disposed therein.

The expression "passageway" as used for the purpose of this invention, includes aperture, orifice, bore, pore, porous element through which the opioid can be pumped, diffuse or migrate through a fiber, capillary tube, porous overlay, porous insert, microporous member, or porous composition. The passageway can also include a compound that erodes or is leached from the wall in the fluid environment of use to produce at least one passageway. Representative compounds for forming a passageway include erodible poly(glycolic) acid, or poly(lactic) acid in the wall; a gelatinous filament; a water-removable poly(vinyl alcohol); leachable compounds such as fluid-removable pore-forming polysaccharides, acids, salts or oxides. A passageway can be formed by leaching a compound from the wall, such as sorbitol, sucrose, lactose, maltose, or fructose, to form a sustained-release dimensional pore-passageway. The passageway can have any shape, such as round, triangular, square and elliptical, for assisting in the sustained metered release of the at least one opioid from the dosage form. The dosage form can be manufactured with one or more passageway in spaced-apart relation on one or more surfaces of the dosage form. A passageway and equipment for forming a passageway are disclosed in U.S. Patent Nos. 3,845,770; 3,916,899; 4,063,064 and 4,088,864. Passageways comprising sustained-release dimensions sized, shaped and adapted as a releasing-pore formed by aqueous leaching to procide a releasing-pore of a sustained-release rate are disclosed in U.S. Patent Nos. 4,200,098 and 4,285,987.

In certain embodiments, the bilayer core comprises a drug layer with the at least one opioid and a displacement or push layer. In certain embodiments the drug layer may also comprise at least one polymer hydrogel. The polymerhydrogel may have an average molecular weight of between about 500 and about 6,000,000. Examples of polymer hydrogels include a maltodextrin polymer comprising the formula (C₆ H₁₂ O₅)ₙ · H₂O, wherein n is 3 to 7,500, and the maltodextrin polymer comprises a 500 to 1,250,000 number-average molecular weight; a poly(alkylene oxide) represented by, e.g., a poly(ethylene oxide) and a poly(propylene oxide) having a 50,000 to 750000 weight-average molecular weight, and more specifically represented by a poly(ethylene oxide) of at least one of 100,000, 200,000, 300,000 or 400,000 weight-average molecular weights; an alkali carboxyalkylcellulose, wherein the alkali is sodium or potassium, the alkyl is methyl, ethyl, propyl, or butyl of 10,000 to 175,000 weight-average molecular weight; and a copolymer of ethylene-acrylic acid, including methacrylic and ethacrylic acid of 10,000 to 500,000 number-average molecular weight.

In certain embodiments of the present invention, the delivery or push layer comprises an osmopolymer. Examples of an osmopolymer include a member selected from the group consisting of a polyalkylene oxide and a carboxyalkylcellulose. The polyalkylene oxide possesses a 1,000,000 to 10,000,000 weight-average molecular weight. The polyalkylene oxide may be a member selected form the group consisting of polymethylene oxide, polyethylene oxide, polypropylene oxide, comprising a 5,000,000 average molecular weight, polyethylene oxide comprising a 7,000,000 average molecular weight, cross-linked polymethylene oxide possessing a 1,000,000 average molecular weight, and polypropylene oxide of 1,200,000 average molecular weight. Typical osmopolymer carboxyalkylcellulose comprises a member selected from the group consisting of alkali carboxyalkylcellulose, sodium carboxymethylcellulose, potassium carboxymethylcellulose, sodium carboxyethylcellulose, lithium carboxymethylcellulose, sodium carboxyethylcellulose, carboxyalkylhydroxyalkylcellulose, carboxymethylhydroxyethyl cellulose, carboxyethylhydroxyethylcellulose and carboxymethylhydroxypropylcellulose. The osmopolymers used for the displacement layer exhibit an osmotic pressure gradient across the semipermeable wall. The osmopolymers imbibe fluid into dosage form, thereby swelling and expanding as an osmotic hacrogel (also known as osmogel), whereby they push the at least one opioid from the osmotic dosage form.

The push layer may also include one or more osmotically effective compounds also known as osmagents and as osmotically effective solutes. They imbibe an environmental fluid, for example, form the gastrointestinal tract, into dosage form and contribute to the delivery kinetics of the displacement layer. Examples of osmotically active compounds comprise a member selected form the group consisting of osmotic salts and osmotic carbohydrates. Examples of specific osmagents include sodium chloride, potassium chloride, magnesium sulphate, lithium phosphate, lithium chloride, sodium phosphate, potassium sulphate, potassium phosphate, glucose, fructose and maltose.

The push layer may optionally include a hydroxypropylalkylcellulose possessing a 9,000 to 450,000 number-average molecular weight. The hydroxypropylalkylcellulose is represented by a member selected from the group consisting of hydroxypropylmethylcellulose, hydroxypropylethylcellulose, hydroxypropylisopropylcellulose, hydroxypropylbutylcellulose and hydroxypropylpentylcellulose.

The push layer optionally may comprise a non-toxic colorant or dye. Examples of colorants or dyes include Food and Drug Administration Colorant (FD&C), such as FD&C No. 1 blue dye, FD&C No. 4 red dye, red ferric oxide, yellow ferric oxide, titanium dioxide, carbon black, and indigo.

The push layer may also optionally comprise an antioxidant to inhibit the oxidation of ingredients. Some examples of antioxidants include a member selected from the group consisting of ascorbic acid, ascorbyl palmitate, butylated hydroxyanisole, a mixute of 2 and 3 tertiary-butyl-4-hydroxyanisole, butylated hydroxytoluene, sodium isoascorbate, dihydroguaretic acid, potassium sorbate, sodium bisulphate, sodium meta bisulphate, sorbic acid, potassium ascorbate, vitamin E, 4-chloro-2,6-ditertiary butylphenol, alpha tocopherol, and propylgallate.

In certain alternative embodiments, the dosage form comprises a homogenous core comprising the opioid, a pharmaceutically acceptable polymer (e.g., polyethylene oxide), optionally a disintegrant (e.g., polyvinylpyrrolidone), optionally an absorption enhancer (e.g., a fatty acid, a surfactant, a chelating agent, a bile salt, ). The homogenous core is surrounded by a semipermeable wall having a passageway (as defined above) for the release of the at least one opioid.

In certain embodiments, the semipermeable wall comprises a member selected from the group consisting of a cellulose ester polymer, a cellulose ether polymer and a cellulose ester-ether polymer. Representative wall polymers comprise a member seleceted from the group consisting of cellulose acylate, cellulose diacylate, cellulose triacylate, cellulose acetate, cellulose diacetate, cellulose triacetate, mono-, di- and tricellulose alkenylates, and mono-, di and tricellulose alkinylates. The poly(cellulose) used for the present invention comprises a number-average molecular weight of 20,000 to 7,500,000.

Additional sempermeable polymers for the purpose of this invention comprise acetaldehyde dimethylcellulose acetate, cellulose acetate ethylcarbamate, cellulose acetate methylcarbamate, cellulose diacetate, propylcarbamate, cellulose acetate diethylaminoacetate; semipermeable polyamide; semipermeable polyurethane; semipermeable sulfonated polystyrene; semipermeable cross-linked polymer formed by the coprecipitation of a polyanion and a polycation as disclosed in U.S. Patent Nos. 3,173,876; 3,276,586; 3,541,005; 3,541,006 and 3,546,876; semipermeable polymers as disclosed by Loeb and Sourirajan in U.S. Patent No. 3,133,132; semipermeable crosslinked polystyrenes; semipermeable crosslinked (poly(sodium styrene sulfonate); semipermeable crosslinked poly(vinylbenzyltrimethyl ammonium chloride); and semipermeable polymers possessing a fluid permeability of 2.5x10⁻⁸ to 2.5x10⁻² (cm²/hr · atm) expressed per atmosphere of hydrostatic or osmotic pressure difference across the semipermeable wall. Other polymers useful in the present invention are known in the art in U.S. Patent Nos. 3,845,770; 3,916,899 and 4,160,020); and in Handbook of Common Polymers, Scott, J.R. and W.J. Roff, 1971, CRC Press, Cleveland, Ohio.

In certain embodiments, preferably the semipermeable wall is non-toxic, inert, and it maintains its physical and chemical integrity during the dispensing life of the drug. In certain embodiments, the dosage form comprises a binder. An example of a binder includes a therapeutically acceptable vinyl polymer having a 5,000 to 350,000 viscosity-average molecular weight, represented by a member selected from the group consisting of poly-n-vinylamide, poly-n-vinylacetamide, poly(vinylpyrrolidone), also known as poly-n-vinylpyrrolidone), poly-n-vinylcaprolactone, poly-n-vinyl-5-methyl-2-pyrrolidone, and poly-n-vinylpyrrolidone copolymers with a member selected from the group consisting of vinyl acetate, vinyl alcohol, vinyl chloride, vinyl fluoride, vinyl butyrate, vinyl laureate, and vinyl stearate. Other binders include for example, acacia, starch, gelatin, and hydroxypropylalkylcellulose of 9,200 to 250,000 average molecular weight.

In certain embodiments, the dosage form comprises a lubricant, which may be used during the manufacture of the dosage form to prevent sticking to die wall or puch faces. Examples of lubricants include magnesium stearate, sodium stearate, stearic acid, calcium stearate, magnesium oleate, oleic acid, potassium oleate, carprylic acid, sodium stearyl fumarate, and magnesium palmitate.

In certain preferred embodiments, the present invention includes a therapeutic composition comprising 1 to 640 mg of opioid, 25 to 500 mg of poly(alkylene oxide) having a 150,000 to 500,000 average molecular weight 1 to 50 mg of poly(vinylpyrrolidone) having a 40,000 average molecular weight, and 0 to about 7.5 mg of a lubricant.

In certain embodiments, the invention also provides a method for administering at least one opioid by admitting orally a dosage form comprising 1 to 640 mg of opioid, a semipermeable wall permeable to aqueous-biological fluid and impervious to the passageway of the opioid which semipermeable wall surrounds an internal space comprising the opioid composition and a push composition, the opioid composition comprising 1 to 640 mg of opioid , 25 to 500 mg of a poly(alkylene oxide) having a 150,000 to 500,000 average molecular weight, 1 to 50 mg of a poly(vinylpyrrolidone) having a 40,000 average molecular weight, and 0 to 7.5 mg of a lubricant, said push composition comprising 15 to 250 mg of a poly(alkylene oxide) of 3,000,000 to 7,500,000 average molecular weight, 0 to 75 mg of an osmagent, 1 to 50 mg of a hydroxyalkylcellulose, 0 to 10 mg of ferric oxide, 0 to 10 mg of a lubricant, and 0 to 10 mg of antioxidant; and a passageway in the imbibing fluid through the semipermeable wall into the dosage form causing the opioid composition to become dispensable and the push composition to expand and push the opioid composition through the passageway, whereby through the combined operations of the dosage form, the opioid is delivered at a therapeutically effective dose at a rate controlled over a sustained period of time.

The dosage forms of the present invention may optionally be coated with one or more coating suitable for the regulation of release or for the protection of the formulation. In one embodiment, coatings are provided to permit either pH-dependent or pH-independent release, e.g., when exposed to gastrointestinal (GI) fluid. When a pH-independent coating is desired, the coating is designed to achieve optimal release regardless of pH-changes in the environmental fluid, e.g., the GI tract. Other preferred embodiments include a pH-denpendent coating that releases the opioid in desired areas of the GI tract, e.g., the stomach or small intestine, such that an absorption profile is provided which is capable of providing at least about twelve hours and preferably about twenty-four hours or more of a therapeutical effect to a patient. It is also possible to formulate compositions which release a portion of the dose in one desired area of the GI tract, e.g., the stomach, and release the remainder of the dose in another area of the GI tract, e.g., the small intestine.

Formulations according to the invention that utilize pH-dependent coatings may also impart a repeat-action effect whereby unprotected drug is coated over an enteric coat and is released in the stomach, while the remainder, being protected by the enteric coating, is released further down the gastrointestinal tract. Coatings which are pH-dependent and may be used in accordance with the present invention include a sustained release material such as, e.g., shellac, cellulose acetate phthalate (CAP), polyvinyl acetate phthalate (PVAP), hydroxypropyl methylcellulose phthalate, and methacrylic acid ester copolymers and zein.

In certain embodiments of the present invention, an effective amount of opioid in immediate release form is included in the formulation. By including such an effective amount of immediate release opioid in the unit dose. In such embodiments, an effective amount of the opioid in immediate release form may be coated onto the tablet of the present invention. For example, where the extended release of the at least one opioid from the formulation is due to a sustained release coating, the immediate release layer would be overcoated on top of the sustained release coating. On the other hand, the immediate release layer may be coated onto the surface of tablets wherein the at least one opioid is incorporated in a sustained release matrix. One skilled in the art would recognize still other alternative manners of incorporating the immediate release opioid portion into the formulation. Such alternatives are deemed to be encompassed by the appended claims.

The following examples illustrate some preferred preparations.

### Example 1 - Production of tablets with different oxycodone/naloxone amounts in a non-swellable diffusion matrix by spray granulation:

The following amounts of the listed components were used for the production of oxycodone/naloxone tablets.

| | | | |
|---|---|---|---|
| Preparation (designation) | Oxy/Nal-0 | Oxy/Nal-5 | Oxy/Nal-10 |
| Oxycodone HCl | 20.0 mg | 20.0 mg | 20.0 mg |
| Naloxone HCl | - | 5.0 mg | 10.0 mg |
| Lactose Flow Lac 100 | 59.25 mg | 54.25 mg | 49.25 mg |
| Povidone 30 Surelease® | 5.0 mg 10.0 mg solid material | 5.0 mg 10.0 mg solid material | 5.0 mg 10.0 mg solid material |
| Stearyl Alcohol | 25.0 mg | 25.0 mg | 25.0 mg |
| Talcum | 2.5 mg | 2.5 mg | 2.5 mg |
| Mg-Stearate | 1.25 mg | 1.25 mg | 1.25 mg |

The Surelease® E-7-7050 polymer mixture used had the following composition.
Surelease®
Ethylcellulose 20 cps
Dibutylsebacate
Ammoniumhydroxide
Oleic acid
Siliciumdioxide
Water

For the production of tablets oxycodone HCl, naloxone HCl, Povidone 30 and Lactose Flow Lac 100 were mixed in a tumbling mixer (Bohle) and subsequently spray-granulated with Surelease® E-7-7050 in a fluidized bath granulating device (GPCG3). The material was sieved over a Comill 1.4 mm sieve. An additional granulation step was carried out with melted fatty alcohol in a high-shear mixer

(Collette). All tablet cores produced by this approach had a weight of 123 mg, based on dry substance.

### Example 2 - Production of tablets with oxycodone and naloxone in a non-swellable diffusion matrix by extrusion:

The following amounts of the listed components were used for the production of the oxycodone/naloxone tablets according to the invention.

| | |
|---|---|
| Preparation (designation) | Oxy/Nal-Extr |
| Oxycodone HCl | 20 mg |
| Naloxone HCl | 10 mg |
| Kollidon 30 | 6 mg |
| Lactose Flow Lac 100 | 49.25 mg |
| Ethylcellulose 45 cps | 10 mg |
| Stearyl alcohol | 24 mg |
| Talcum | 2.5 mg |
| Mg-Stearate | 1,25 mg |

The listed amounts of oxycodone HCl, naloxone HCl, ethylcellulose 45 cps, Povidone 30, stearyl alcohol and Lactose Flow Lac 100 were mixed in a tumbling mixer (Bohle). This mixture was subsequently extruded with a counter-rotating twin screw extruder of the type Micro 18 GGL (Leistritz AG, Nürnberg, Germany). The temperature of heating zone 1 was 25°C, of heating zone 2, 50°C, of heating zones 3 to 5, 60°C, of heating zones 6 to 8, 55°C, of heating zone 9, 60°C and of heating zone 10, 65°C. The screw rotating speed was 150 revolutions per minute (rpm), the resulting melt temperature was 87°C, the feed rate was 1.5 kg/h and the diameter of the nozzle opening was 3 mm. The extruded material was sieved with a Frewitt 0.68 x 1.00 mm sieve. The grinded extrudate was then mixed with talcum and magnesium stearate that had been added over a 1 mm hand sieve and was subsequently pressed into tablets.

In comparison to the oxycodone/naloxone tablets which also have the Surelease®-based non-swellable diffusion matrix produced by spray granulation (see Example 1), extruded preparations comprise less components.

### Example 3 - Release profile of the oxycodone/naloxone tablets from Example 1:

The release of the active compounds was measured over a time period of 12 hours, applying the Basket Method according to USP at pH 1.2 using HPLC. Tablets Ox/Nal-0, Ox/Nal-5 and Ox/Nal-10 were tested.

One recognizes from the table that in the case of a non-swellable diffusion matrix based on Surelease®, the release rates of different oxycodone amounts, independent of the naloxone amount, remain equal (invariant). Correspondingly, invariant release profiles are observed for naloxone at different oxycodone amounts.

| Time (min) | Ox/Nal-0 | Ox/Nal-5-O | Ox/Nal-5-N | Ox/Nal-10-O N | Ox/Nal-10-N |
|---|---|---|---|---|---|
| | Oxy | Oxy | Nal | Oxy | Nal |
| 0 | 0 | 0 | 0 | 0 | 0 |
| 15 | 26.1 | 24.9 | 23.5 | 22.8 | 24.1 |
| 120 | 62.1 | 63 | 61 | 57.5 | 60.2 |
| 420 | 91.7 | 94.5 | 91.9 | 89.4 | 93.5 |
| 720 | 98.1 | 99.6 | 96.6 | 95.7 | 100.6 |

The release values refer to oxycodone or naloxone (line 2) and are given as percentages. The mean value for the release of naloxone at e. g. 420 min is 92,7%. The maximal deviation at 420 min is 1%. Oxy and Nal stand for oxycodone and naloxone and indicate the active compound which has been measured.

### Example 4 - Release profile of oxycodone/naloxone tablets from Example 2 at different pH-values:

The release of active compounds from the tablets was measured over a time period of 12 hours at pH 1.2 or for 1 hour at 1.2 and subsequently for 11 hours at pH 6.5. Release rates were determined by the basket method according to USP using HPLC.

The following release rates were measured for 12 hours at pH 1.2:

| Time | Oxy/Nal-Extr-1,2-O | Oxy/Nal-Extr-1,2-N |
|---|---|---|
| (min) | Oxy | Nal |
| 0 | 0 | 0 |
| 15 | 24.1 | 24.0 |
| 120 | 62.9 | 63.5 |
| 420 | 92.9 | 93.9 |
| 720 | 96.9 | 98.1 |

The following release rates were measured for 1 hour at pH 1.2 and 11 hours at pH 6.5:

| Time | Oxy/Nal-Extr-6,5-O | Oxy/Nal-Extr-6,5-N |
|---|---|---|
| (min) | Oxy | Nal |
| 0 | 0 | 0 |
| 60 | 48.1 | 49.2 |
| 120 | 65.0 | 64.7 |
| 240 | 83.3 | 81.8 |
| 420 | 94.1 | 92.3 |

The release rates refer to oxycodone and naloxone (line 2) and are given as percentages. Oxy and Nal stand for oxycodone and naloxone and indicate the active compound measured.

Further suitable examples with a combination of oxycodone as agonist and naloxone as antagonist are disclosed in WO 2003084504.

Reference Examples 5 and 6 - Sustained Release Oxycodone formulations, 10 and 20 mg tablets

Eudragite® RS 30D and Triacetine are combined while passing though a 60 mesh screen, and mixed under low shear for approximately 5 minutes or until a uniform dispersion is observed.

Next, suitable quantities of Oxycodone HCl, lactose, and povidone are placed into a fluid bed granulator/dryer (FBD) bowl, and the suspension sprayed onto the powder in the fluid bed. After spraying, the granulation is passed through a #12 screen if necessary to reduce lumps. The dry granulation is placed in a mixer.

In the meantime, the required amount of stearyl alcohol is melted at a temperature of approximately 70°C. The melted stearyl alcohol is incorporated into the granulation while mixing. The waxed granulation is transferred to a fluid bed granulator/dryer or trays and allowed to cool to room temperature or below. The cooled granulation is then passed through a #12 screen. Thereafter, the waxed granulation is placed in a mixer/blender and lubricated with the required amounts of talc and magnesium stearate for approximately 3 minutes, and then the granulate is compressed into 125 mg tablets on a suitable tableting machine.

The formula for the tablets of Example 5 (10 mg tablet) is set forth in the table below:

| Component | Mg/Tablet | %(by wt) |
|---|---|---|
| Oxycodone Hydrochloride | 10.0 | 8.0 |
| Lactose (spray dried) | 69.25 | 55.4 |
| Povidone | 5.0 | 4.0 |
| Eudragit® RS 30D (solids) | 10.0* | 8.0 |
| Triacetin® | 2.0 | 1.6 |
| Stearyl Alcohol | 25.0 | 20.0 |
| Talc | 2.5 | 2.0 |
| Magnesium Stearate | 1.25 | 1.0 |
| Total: | 125.0 | 100.0 |

| | | |
|---|---|---|
| *Approximately 33.33 mg Eudragit® RS 30D Aqueous dispersion is equivalent to 10 mg of Eudragit® RS 30D dry substance. | | |

The formula for the tablets of Example 6 (20 mg tablet) is set forth in the table below:

| Component | Mg/Tablet |
|---|---|
| Oxycodone Hydrochloride | 20.0 |
| Lactose (spray dried) | 59.25 |
| Povidone | 5.0 |
| Eudragit® RS 30D (solids) | 10.0* |
| Triacetin® | 2.0 |
| Stearyl Alcohol | 25.0 |
| Talc | 2.5 |
| Magnesium Stearate | 1.25 |
| Total: | 125.0 |

Reference Example 7- The tablets of Example 5 are then tested for dissolution via the USP Basket Method at 37°C, 100 RPM, first hour 700 ml simulated gastric fluid at pH 1.2, then changed to 900 ml at pH 7.5. The results are set forth in the table below:

| Time (hours) | % Oxycodone Dissolved |
|---|---|
| 1 | 38.0 |
| 2 | 47.5 |
| 4 | 62.0 |
| 8 | 79.8 |
| 12 | 91.1 |
| 18 | 94.9 |
| 24 | 98.7 |

Reference Example 8 - The tablets of Example 6 are then tested for dissolution via the USP Basket Method at 3°C, 100 RPM, first hour 700 ml simulated gastric fluid at pH 1.2, then changed to 900 ml at pH 7.5. The results are set forth in the table below:

| Time (hours) | % Oxycodone Dissolved |
|---|---|
| 1 | 31 |
| 2 | 44 |
| 4 | 57 |
| 8 | 71 |
| 12 | 79 |
| 18 | 86 |
| 24 | 89 |

Further suitable examples with oxycodone as agonist and corresponding in vivo data are disclosed in EP 0 576 643

Reference Example 9 - 24 hour 160 mg oxycodone sustained release capsules were prepared with the formula set forth in table below:

| Component | Mg/unit |
|---|---|
| Oxycodone HCL | 160 |
| Stearic Acid | 80 |
| Stearyl Alcohol | 20 |
| Eudragit RSPO | 140 |
| Total | 400 |

The formulation above was prepared according to the following procedure:
1. Pass the stearyl alcohol flakes through an impact mill.
2. Blend the Oxycodone HCl, stearic acid, stearyl alcohol and the Eudragit RSPO in a suitable lender/mixer.
3. Continuously feed the blended material into a twin screw extruder at elevated temperatures and collect the resultant strands on a conveyor.
4. Allow the strands to cool on the conveyor.
5. Cut the strands into 1 mm pellets using a pelletizer.
6. Screen the pellets for fines and oversized pellets to an acceptable range of about 0.8
   1.4 mm in size
7. Fill into capsules with a fill weight of 400 mg/capsule (Fill into size 00 capsules).

Reference Example 10 - The tablets of Example 9 are then tested for dissolution The pellets were then using the following procedure:
Fiber optic UV dissolution using USP apparatus 1 (basket) at 100 rpm in 900 ml simulated gastric fluid (SGF) and in 900 ml simulated intestinal fluid (SIF) monitoring at 282nm.
The dissolution parameters for the above formulation are set forth in Table below:.

| Time (hour) | %Dissolved in SGF | % Dissolved in SIF |
|---|---|---|
| 1 | 32 | 20 |
| 2 | 47 | 28 |
| 4 | 66 | 42 |
| 8 | 86 | 60 |
| 12 | 93 | 70 |
| 18 | 95 | 77 |
| 24 | 95 | 80 |

## Claims

1. Use of an opioid sustained release oral dosage form comprising a mixture of an opioid agonist selected from the group comprising oxycodon, hydrocodone, hydromorphone, morphine, methadone, oxymorphone, fentanyl and sufentanyl, in the form of the free base or a pharmaceutically acceptable salt, and an opioid antagonist selected from the group comprising naltrexone, nalmefene and naloxone, in the form of the free base or a pharmaceutically acceptable salt, for the manufacture of a medicament to treat patients with restless leg syndrome (RLS).

2. Use of an opioid sustained release oral dosage form comprising a mixture of an opioid agonist selected from the group comprising oxycodon, hydrocodone, hydromorphone, morphine, methadone, oxymorphone, fentanyl and sufentanyl, in the form of the free base or a pharmaceutically acceptable salt, and an opioid antagonist selected from the group comprising naltrexone, nalmefene and naloxone, in the form of the free base or a pharmaceutically acceptable salt, for the manufacture of a medicament to treat patients with restless leg syndrome (RLS) which provides an effective treatment when administered every 12 hours at steady state.

3. Use of an opioid sustained release oral dosage form comprising a mixture of an opioid agonist selected from the group comprising oxycodon, hydrocodone, hydromorphone, morphine, methadone, oxymorphone, fentanyl and sufentanyl, in the form of the free base or a pharmaceutically acceptable salt, and an opioid antagonist selected from the group comprising naltrexone, nalmefene and naloxone, in the form of the free base or a pharmaceutically acceptable salt, for the manufacture of a medicament to treat patients with restless leg syndrome (RLS) which provides an effective treatment when administered every 24 hours at steady state.

4. Use according to any preceding claim, wherein the oral dosage form contains oxycodone or a pharmaceutically acceptable salt thereof.

5. Use according to claims 1 to 3, wherein the oral dosage form comprises morphine or a pharmaceutically acceptable salt thereof.

6. Use according to claims 1 to 4, wherein the oral dosage form comprises a mixture of oxycodone and naloxone, in the form of the free base or as pharmaceutically acceptable salts thereof.

7. Use according to claim 6, wherein the oral dosage form is a storage stable pharmaceutical preparation and the active compounds are released from the preparation in a sustained, invariant and independent manner.

8. Use according to claim 6 or 7, wherein oxycodone is present in excess referred to the unit dosage amount of naloxone.

9. Use according to claims 6 to 8, wherein naloxone is present in an amount range of 1 to 50 mg.

10. Use according to claims 6 to 9, wherein oxycodone is present in an amount range of 10 to 150 mg, preferably of 10 to 80 mg.

11. Use according to claims 6 to 10, wherein oxycodone and naloxone are present in weight ratio ranges of maximal 25 : 1, preferably of maximal 20:1, 15:1, especially preferably of 5:1, 4:1, 3:1, 2:1 or 1:1.

## Patentansprüche

1. Opioid-lang anhaltend freisetzende orale Dosierungsform umfassend eine Mischung eines Opioid-Agonisten ausgewählt aus der Gruppe umfassend Oxycodon, Hydrocodon, Hydromorphon, Morphin, Methadon, Oxymorphon, Fentanyl und Sufentanyl, in Form der freien Base oder eines pharmazeutisch verträglichen Salzes, und eines Opioid-Antagonisten ausgewählt aus der Gruppe umfassend Naltrexon, Nalmefen und Naloxon, in Form der freien Base oder eines pharmazeutisch verträglichen Salzes, zur Verwendung bei der Behandlung von Patienten mit dem Restless-Legs-Syndrom (RLS; im Deutschen: "Syndrom der unruhigen Beine").

2. Opioid-lang anhaltend freisetzende orale Dosierungsform umfassend eine Mischung eines Opioid-Agonisten ausgewählt aus der Gruppe umfassend Oxycodon, Hydrocodon, Hydromorphon, Morphin, Methadon, Oxymorphon, Fentanyl und Sufentanyl, in Form der freien Base oder eines pharmazeutisch verträglichen Salzes, und eines Opioid-Antagonisten ausgewählt aus der Gruppe umfassend Naltrexon, Nalmefen und Naloxon, in Form der freien Base oder eines pharmazeutisch verträglichen Salzes, zur Verwendung bei der Behandlung von Patienten mit dem Restless-Legs-Syndrom (RLS; im Deutschen: "Syndrom der unruhigen Beine"), die eine wirksame Behandlung bei einer Verabreichung alle 12 Stunden im Fließgleichgewicht zur Verfügung stellt.

3. Opioid-lang anhaltend freisetzende orale Dosierungsform umfassend eine Mischung eines Opioid-Agonisten ausgewählt aus der Gruppe umfassend Oxycodon, Hydrocodon, Hydromorphon, Morphin, Methadon, Oxymorphon, Fentanyl und Sufentanyl, in Form der freien Base oder eines pharmazeutisch verträglichen Salzes, und eines Opioid-Antagonisten ausgewählt aus der Gruppe umfassend Naltrexon, Nalmefen und Naloxon, in Form der freien Base oder eines pharmazeutisch verträglichen Salzes, zur Verwendung bei der Behandlung von Patienten mit dem Restless-Legs-Syndrom (RLS; im Deutschen: "Syndrom der unruhigen Beine"), die eine wirksame Behandlung bei einer Verabreichung alle 24 Stunden im Fließgleichgewicht zur Verfügung stellt.

4. Opioid-lang anhaltend freisetzende orale Dosierungsform zur Verwendung gemäß eines jeden der vorangegangenen Ansprüche, wobei die orale Dosierungsform Oxycodon oder ein pharmazeutisch verträgliches Salz davon enthält.

5. Opioid-lang anhaltend freisetzende orale Dosierungsform zur Verwendung gemäß den Ansprüchen 1 bis 3, wobei die orale Dosierungsform Morphin oder ein pharmazeutisch verträgliches Salz davon umfasst.

6. Opioid-lang anhaltend freisetzende orale Dosierungsform zur Verwendung gemäß den Ansprüchen 1 bis 4, wobei die orale Dosierungsform eine Mischung von Oxycodon und Naloxon, in Form der freien Base oder des pharmazeutisch verträglichen Salzes davon, umfasst.

7. Opioid-lang anhaltend freisetzende orale Dosierungsform zur Verwendung gemäß Anspruch 6, wobei die orale Dosierungsform eine lagerstabile pharmazeutische Präparation ist und die Wirkstoffe von der Präparation in einer lang anhaltenden, unveränderlichen und unabhängigen Art und Weise freigesetzt werden.

8. Opioid-lang anhaltend freisetzende orale Dosierungsform zur Verwendung gemäß Anspruch 6 oder 7, wobei Oxycodon bezogen auf die Einheitsdosierungsmenge von Naloxon im Überschuss vorhanden ist.

9. Opioid-lang anhaltend freisetzende orale Dosierungsform zur Verwendung gemäß den Ansprüchen 6 bis 8, wobei Naloxon in einem Mengenbereich von 1 bis 50 mg vorhanden ist.

10. Opioid-lang anhaltend freisetzende orale Dosierungsform zur Verwendung gemäß den Ansprüchen 6 bis 9, wobei Oxycodon in einem Mengenbereich von 10 bis 150 mg, bevorzugt von 10 bis 80 mg vorhanden ist.

11. Opioid-lang anhaltend freisetzende orale Dosierungsform zur Verwendung gemäß den Ansprüche 6 bis 10, wobei Oxycodon und Naloxon in Gewichtsverhältnis-Bereichen von maximal 25 : 1, bevorzugt von maximal 20 : 1, 15 : 1, besonders bevorzugt von 5 : 1, 4 : 1, 3 : 1, 2 : 1 oder 1 : 1 vorhanden sind.

## Revendications

1. Forme posologique orale à libération prolongée d'opioïde, comprenant un mélange d'un agoniste d'opioïde choisi dans le groupe comprenant l'oxycodone, l'hydrocodone, l'hydromorphone, la morphine, la méthadone, l'oxymorphone, le fentanyl et le sufentanyl, sous forme de la base libre ou d'un sel pharmaceutiquement acceptable, et d'un antagoniste d'opioïde choisi dans le groupe comprenant la naltrexone, le nalméfène et la naloxone, sous forme de la base libre ou d'un sel pharmaceutiquement acceptable, pour son utilisation dans le traitement de patients atteints du syndrome des jambes sans repos (RLS).

2. Forme posologique orale à libération prolongée d'opioïde, comprenant un mélange d'un agoniste d'opioïde choisi dans le groupe comprenant l'oxycodone, l'hydrocodone, l'hydromorphone, la morphine, la méthadone, l'oxymorphone, le fentanyl et le sufentanyl, sous forme de la base libre ou d'un sel pharmaceutiquement acceptable, et d'un antagoniste d'opioïde choisi dans le groupe comprenant la naltrexone, le nalméfène et la naloxone, sous forme de la base libre ou d'un sel pharmaceutiquement acceptable, pour son utilisation dans le traitement de patients atteints du syndrome des jambes sans repos (RLS), qui assure un traitement efficace lors de l'administration toutes les 12 heures en régime constant.

3. Forme posologique orale à libération prolongée d'opioïde, comprenant un mélange d'un agoniste d'opioïde choisi dans le groupe comprenant l'oxycodone, l'hydrocodone, l'hydromorphone, la morphine, la méthadone, l'oxymorphone, le fentanyl et le sufentanyl, sous forme de la base libre ou d'un sel pharmaceutiquement acceptable, et d'un antagoniste d'opioïde choisi dans le groupe comprenant la naltrexone, le nalméfène et la naloxone, sous forme de la base libre ou d'un sel pharmaceutiquement acceptable, pour son utilisation dans le traitement de patients atteints du syndrome des jambes sans repos (RLS), qui assure un traitement efficace lors de l'administration toutes les 24 heures en régime constant.

4. Forme posologique orale pour son utilisation suivant l'une quelconque des revendications précédentes contenant de l'oxycodone ou un de ses sels pharmaceutiquement acceptables.

5. Forme posologique orale pour son utilisation suivant les revendications 1 à 3 comprenant de la morphine ou un de ses sels pharmaceutiquement acceptables.

6. Forme posologique orale pour son utilisation suivant les revendications 1 à 4 comprenant un mélange d'oxycodone et de naloxone, sous forme de la base libre ou de leurs sels pharmaceutiquement acceptables.

7. Forme posologique orale pour son utilisation suivant la revendication 6, dans laquelle la forme posologique orale est une préparation pharmaceutique stable au stockage et les composés actifs sont libérés de la préparation d'une manière prolongée, invariante et indépendante.

8. Forme posologique orale pour son utilisation suivant la revendication 6 ou 7, dans laquelle l'oxycodone est présente en un excès établi par rapport à la quantité posologique unitaire de naloxone.

9. Forme posologique orale pour son utilisation suivant les revendications 6 à 8, dans laquelle la naloxone est présente en une quantité comprise dans l'intervalle de 1 à 50 mg.

10. Forme posologique orale pour son utilisation suivant les revendications 6 à 9, dans laquelle l'oxycodone est présente en une quantité comprise dans l'intervalle de 10 à 150 mg, de préférence de 10 à 80 mg.

11. Forme posologique orale pour son utilisation suivant les revendications 6 à 10, dans laquelle l'oxycodone et la naloxone sont présentes dans des intervalles de rapport pondéral au maximum de 25:1, de préférence au maximum de 20:1, de 15:1, notamment de 5:1, 4:1, 3:1, 2:1 ou 1:1.
